# EUROPÄISCHE PATENTANMELDUNG

(11) **EP 4 806 878 A1**
(43) Veröffentlichungstag der Anmeldung: **16.09.2026**
(21) Anmeldenummer: 25162513.3
(22) Anmeldetag: 10.03.2025
(51) Int. Cl.: C07C 41/58, C07C 43/04, C07C 1/20, C07C 11/09, C07C 7/04, C07C 29/10, C07C 31/04, C07C 29/80

(54) **ENERGIEEFFIZIENTES VERFAHREN ZUR SPALTUNG VON METHYL-TERT.-BUTYL-ETHER (MTBE)**

(71) Anmelder: Evonik Oxeno GmbH & Co. KG, 45772 Marl (DE)
(72) Erfinder: PAUL, Niklas, 45770 Marl (DE); RIX, Armin Matthias, 45770 Marl (DE); WESSNER, Lea, 44139 Dortmund (DE); SIX, Tanita Valèrie, 44309 Dortmund (DE); STEENWEG, Tanja, 44139 Dortmund (DE); OLDEMEYER, Martin, 45721 Haltern am See (DE); WÜLLER, Martin, 45768 Marl (DE); FABER, Kai-Uwe, 45721 Haltern am See (DE); KNOSSALLA, Johannes, 48351 Everswinkel (DE)
(74) Vertreter: Evonik Patent Association

(57) **Zusammenfassung**

Gegenstand der Erfindung ist ein Verfahren zur Spaltung von MTBE, bei dem das aus dem bereitgestellten Feedstrom erhaltene MTBE einer Spaltungsreaktion unterworfen und das entstandene Isobuten anschließend abgetrennt wird. Das erfindungsgemäße Verfahren zeichnet sich dadurch aus, dass Energie während des Verfahrens zurückgewonnen und innerhalb des Verfahrens eingesetzt wird.

## Beschreibung

Die vorliegende Erfindung betrifft ein Verfahren zur Spaltung von MTBE, bei dem das aus dem bereitgestellten Feedstrom erhaltene MTBE einer Spaltungsreaktion unterworfen und das entstandene Isobuten anschließend abgetrennt wird. Das erfindungsgemäße Verfahren zeichnet sich dadurch aus, dass Energie während des Verfahrens zurückgewonnen und innerhalb des Verfahrens eingesetzt wird.

Isobuten ist Ausgangsstoff für die Herstellung einer Vielzahl von Produkten, beispielsweise für die Herstellung von Butylkautschuk, Polyisobutylen, Isobuten-Oligomeren und t-Butylaromaten. Darüber hinaus kann Isobuten als Vorstufe für die Herstellung von Methacrylsäure und deren Ester verwendet werden.

In technischen Strömen wie C4-Schnitten aus Erdölcrackern liegt Isobuten häufig zusammen mit gesättigten und ungesättigten C4-Kohlenwasserstoffen vor. Aus diesen Gemischen kann Isobuten wegen des geringen Siedepunktsunterschieds zwischen Isobuten und 1-Buten durch Destillation nur schwer abgetrennt werden. Daher wird Isobuten aus technischen Kohlenwasserstoffgemischen üblicherweise dadurch gewonnen, dass Isobuten mit Methanol zu MTBE umgesetzt wird, das sich leicht vom übrig gebliebenen Kohlenwasserstoffgemisch abtrennen lässt und dadurch dass das isolierte MTBE anschließend zu Isobuten zurück gespalten wird.

Ausgehend von dem aus der MTBE-Synthese erhaltenen Feedstrom umfasst das Verfahren verschiedene Trenn- bzw. Destillationsschritte und die Spaltungsreaktion an sich. Die Trenn- bzw. Destillationsschritte haben einen hohen Energiebedarf, um die Trennung in ausreichendem Maße gewährleisten zu können. Die Energie wird hauptsächlich über Dampf eingebracht, was aufgrund der großen Dampfmengen erhebliche Kosten verursacht und gleichzeitig hohe CO₂-Emmisionen erzeugt.

Es bestand daher die Aufgabe, einen Prozess Spaltung von MTBE zu entwickeln, bei dem die für die Trennung bzw. Destillation notwendige Menge an Dampf reduziert werden kann, um dadurch sowohl Kosten einzusparen als auch die CO₂-Emissionen zu reduzieren.

Die Aufgabe kann durch das erfindungsgemäße Verfahren gemäß Anspruch 1 gelöst werden. Bevorzugte Ausführungsformen sind in den abhängigen Ansprüchen angegeben.

Das erfindungsgemäß Verfahren ist ein Verfahren zur Spaltung von MTBE, wobei das Verfahren zumindest die folgenden Schritte aufweist:
a) Bereitstellen eines Feedstroms, der MTBE enthält;
b) Leichtsiederabtrennung aus dem in Schritt (a) bereitgestellten Feedstrom in einer Destillationskolonne DL, wobei am Kopf ein Strom LK anfällt und der Sumpfstrom LS zum nachfolgenden Schritt (c) geführt wird;
c) Destillative Abtrennung des MTBE aus dem Sumpfstrom LS in mindestens einer Destillationskolonnen DM, wobei am Kopf von DM ein Strom K anfällt, der zumindest MTBE enthält, wobei der Strom K teilweise kondensiert wird, wodurch Energie auf einen Wärmeträger WA übertragen und ein Dampfstrom DS1 erzeugt wird, und teilweise zur nachfolgenden Spaltungsreaktion in Schritt (d) geführt wird;
d) Durchführen der Spaltungsreaktion von MTBE mit dem Strom K in der Gasphase in mindestens einem Reaktor unter Verwendung eines heterogenen Katalysators, wodurch ein Reaktionsgemisch R erhalten wird, das zumindest Isobuten, Methanol, Wasser und MTBE enthält, wobei der Strom K vor dem Eintritt in den mindestens einen Reaktor und/oder der mindestens eine Reaktor mit einem Wärmeträgerfluid beheizt wird, das über einen Wärmeträgerfluidkreislauf bereitgestellt wird;
e) Destillation des Reaktionsgemischs R in mindestens einer Destillationskolonne DR, wobei ein Strom IB, der zumindest Isobuten enthält, und ein Strom M, der zumindest Methanol und Wasser enthält, anfallen;
f) Wasserabtrennung aus Strom M in einer einzigen Destillationskolonne DW1, wobei am Kopf der Destillationskolonne DW1 ein Strom WK, der zumindest Methanol enthält, und am Sumpf ein Strom, der zumindest Wasser enthält, anfällt, wobei der Strom WK einer ein- oder mehrstufigen Brüdenverdichtung unterworfen und dann zur Beheizung der Destillationskolonne DW1 eingesetzt wird;
g) Zumindest teilweises Kondensieren des Kopfstroms LK, wobei die Kondensationswärme dadurch genutzt wird, dass Energie vom Kopfstrom LK auf einen flüssigen oder gasförmigen Wärmeträger W übertragen wird, wodurch ein Wärmeträger W1 entsteht;
h) Zumindest teilweises Kondensieren des Reaktionsgemisches R, wobei die Kondensationswärme dadurch genutzt wird, dass Energie vom Reaktionsgemisch R auf einen flüssigen oder gasförmigen Wärmeträger W übertragen wird, wodurch ein Wärmeträger W2 entsteht;
i) Vermischen der beiden Wärmeträger W1 und W2 unter Erhalt des Wärmeträgers W3;
j) Verdichten mindestens eines Teils des Wärmeträgers W3, vorzugsweise des gesamten Wärmeträgers W3, wodurch ein verdichteter Wärmeträger W4 entsteht, der einen höheren Druck aufweist als der Wärmeträger W3; und
k) Übertragen von Energie vom verdichteten Wärmeträger W4 auf einen weiteren Wärmeträger WB übertragen wird, wodurch ein Dampfstrom DS2 erzeugt wird, der teilweise zur Beheizung der Destillationskolonne DL, der Destillationskolonne DR und optional der Destillationskolonne DW1 verwendet wird und teilweise mit dem Dampfstrom DS1 vermischt, unter Erhalt eines verdichteten Dampfstroms DS3 verdichtet und zur Beheizung der Destillationskolonne DM und nach weiterer Verdichtung unter Erhalt eines weiter verdichteten Dampfstroms DS4 zur Beheizung des Wärmeträgerfluidkreislaufs eingesetzt wird.

Kennzeichnend für das Verfahren ist, dass die Kondensationsenergie der Kopfströme, die bei der Leichtsiederabtrennung in Schritt b) und bei der Wasserabtrennung in Schritt f) anfallen, mittels eines Wärmeträger nutzbar gemacht wird und in verschiedenen Destillationskolonnen zur Beheizung eingesetzt wird. Die dort jeweils darüber eingespeiste Wärmemenge muss dementsprechend nicht mehr über Dampf eingebracht werden. Dadurch kann Dampf eingespart werden, was zu einer Kostenverringerung und einer Reduzierung der CO₂-Emissionen führt.

### Schritt a)

Der erste Schritt a) betrifft die Bereitstellung eines MTBE-Stroms. Die genaue Zusammensetzung des Stroms ist ebenso wie die Herkunft des Stroms zweitranig, solange der Feedstrom MTBE enthält. Es versteht sich, dass die Mengen an MTBE ausreichend hoch sein müssen, um das Verfahren wirtschaftlich durchzuführen. In der Regel stammt der Feedstrom aus einer MTBE-Synthese

Die MTBE-Synthese ist dem Fachmann grundsätzlich bekannt. Dabei werden Isobuten-haltige C4-Kohlenwasserstoffe wie HCC4 (hydriertes Crack C4), Raffinat I oder Mischungen davon eingesetzt. Das in den Isobuten-haltigen C4-Kohlenwasserstoffen enthaltene Isobuten wird bei der MTBE-Synthese mit Methanol an einem Katalysator zu MTBE umgesetzt.

Als Katalysatoren werden in der MTBE-Synthese vorzugsweise feste saure Ionenaustauscherharze, die Sulfonsäuregruppen aufweisen, eingesetzt. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Das Porenvolumen der als Katalysatoren eingesetzten Ionenaustauscherharze beträgt vorzugsweise von 0,3 bis 0,9 ml/g, insbesondere von 0,5 bis 0,9 ml/g. Die Korngröße der Harze beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauschers beträgt, bezogen auf die Lieferform, bevorzugt von 0,7 bis 2,0 eq/l, insbesondere von 1,1 bis 2,0 eq/l bzw. vorzugsweise von 0,5 bis 5,5 mol/kg, insbesondere von 0,8 bis 5,5 mol/kg.

Die Angaben zur Kapazität in mol/kg beziehen sich auf das jeweils bis zur Gewichtskonstanz im warmen Stickstoffstrom bei z. B. 105 °C getrocknete Ionentauscherharz.

Die MTBE-Synthese kann in einem oder mehreren in Reihe geschalteten Reaktoren stattfinden. Der Katalysator wird vorzugsweise als Festbettkatalysator eingesetzt. Da die Bildung von MTBE eine Gleichgewichtsreaktion darstellt, kann es zweckmäßig sein mindestens eine Reaktivdestillationskolonne einzusetzen, in der gleichzeitig die Reaktion und die Abtrennung des MTBE erfolgen. Bei der Reaktivdestillation sollte ein Druck im Bereich von 3 bis 15 bar und eine Temperatur in der Reaktionszone von 55 bis 75 °C vorliegen.

### Schritt b)

Schritt b) des erfindungsgemäßen Verfahrens werden Leichtsieder von dem im Schritt a) bereitgestellten Feedstrom abgetrennt. Die Abtrennung erfolgt in einer Destillationskolonne DL, wobei am Kopf ein Strom LK und am Sumpf der Sumpfstrom LS anfällt. Der Sumpfstrom LS wird dann zum nachfolgenden Schritt c) geführt wird.

Die Leichtsiederabtrennung kann im einfachsten Fall in einer einzigen Destillationskolonne DL erfolgen. Das Destillat, also der Kopfstrom LK, umfasst insbesondere nicht umgesetzte C4-Kohlenwasserstoffe, die aufgrund der Azeotropbildung mit Methanol auch Methanol enthalten, und Dimethylether (DME). Das Sumpfprodukt LS kann neben dem MTBE hochsiedende Nebenprodukte enthalten. Abhängig vom gewählten Verhältnis Methanol : Isobuten bei der MTBE-Synthese und dem erreichten Umsatz kann auch noch Methanol im Sumpfstrom LS enthalten sein.

Die Destillationskolonne DL weist vorzugsweise 15 bis 55 theoretische Trennstufen auf, bevorzugt 20 bis 40 theoretische Trennstufen auf und besonders bevorzugt 25 bis 35 theoretische Trennstufen auf. Der Zulauf der Kolonne wird vorzugsweise zwischen Stufe 10 und 30 (von oben) zugegeben, bevorzugt zwischen Stufe 15 und 25. Die Kolonne wird vorzugsweise bei einem Druck von 3 bis 25 bar absolut, bevorzugt bei 4 bis 10 bar absolut betrieben. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 2. Als Rücklaufverhältnis bezeichnet man definitionsgemäß im Rahmen der vorliegenden Erfindung das das Verhältnis Massenstrom des Rücklaufs geteilt durch den Massenstrom des Destillats.

### Schritt c)

Auf den Schritt b) folgt die destillative Abtrennung des MTBE aus dem Sumpfstrom LS in Schritt c). Die Abtrennung erfolgt in mindestens einer Destillationskolonnen DM, wobei am Kopf von DM ein Strom K anfällt, wobei der Strom K teilweise kondensiert wird, wodurch Energie auf einen Wärmeträger WA übertragen und ein Dampfstrom DS1 erzeugt wird und teilweise zur nachfolgenden Spaltungsreaktion in Schritt (d) geführt wird.

Das Sumpfprodukt LS aus Schritt a) wird in der in mindestens einer Destillationskolonnen DM weiter aufgereinigt. Dabei können Schwersieder (z. B. Diisobuten oder Methyl-sec-butyl-ether (MSBE)) als Sumpfprodukt entfernt. Eine weitere Aufgabe dieser Kolonne kann die teilweise oder vollständige Abtrennung von 2-Methoxybutan sein, denn 2-Methoxybutan würde in der nachfolgenden Spaltungsreaktion zu linearem Buten und Methanol gespalten werden. Lineare Butene können bei zu hoher Konzentration gegebenenfalls die Isobuten-Spezifikation gefährden.

Die mindestens eine Destillationskolonnen DM wird bei einem Druck betrieben, bei dem möglichst den Großteil der Schwersieder im Sumpf verbleibt und ein möglichst reines MTBE als Destillat anfällt. Der Kolonnendruck liegt daher vorzugsweise bei 3 bis 20 bar absolut. Wenn die Spaltung des im Strom K enthaltenen MTBE im sich anschließenden Spaltungsschritt d bei erhöhtem Druck erfolgt, kann es vorteilhaft sein, die vorliegende Destillation in Schritt c) bei höherem Druck durchzuführen, wobei in diesem Fall der Kopfstrom K dampfförmig abgezogen wird. Das dampfförmig abgezogene Kopfprodukt kann dann direkt oder nach weiterer Vorwärmung dem Reaktor zugeführt werden.

Die mindestens eine Destillationskolonnen DM weist vorzugsweise 15 bis 60 theoretische Trennstufen, weiterhin bevorzugt 20 bis 55 theoretische Trennstufen, besonders bevorzugt 30 bis 45 theoretische Trennstufen auf. Das Rücklaufverhältnis, wie oben definiert, wird, in Abhängigkeit von der realisierten Stufenzahl, der Zusammensetzung des eingesetzten MTBE und der erforderlichen Reinheit, vorzugsweise auf einen Wert von 0,5 bis 7, bevorzugt von 1 bis 5 eingestellt.

Das Sumpfprodukt der mindestens einen Destillationskolonnen DM kann wie erwähnt Schwersieder wie Diisobuten oder 2-Methoxybutan enthalten. Es wird vorzugsweise verwertet, beispielsweise als Einsatzstoff für eine Synthesegasanlage oder ggf. nach einer Hydrierung als Treibstoffkomponente eingesetzt.

Der in der Destillationskolonne DM in Schritt c) anfallende Strom enthält das MTBE, das im nachfolgenden Schritt d) der Spaltungsreaktion unterworfen wird. Der Strom K fällt am Kopf der Destillationskolonne an und wird teilweise zur Wärmeintegration eingesetzt. Der restliche Teil wird zur nachfolgenden Spaltungsreaktion in Schritt d) geführt.

Ein Teil des Stroms K wird nämlich in Schritt c) kondensiert. Die freiwerdende Kondensationsenergie wird benutzt, um einen Wärmeträger WA zu verdampfen, wodurch ein Dampfstrom DS1 erzeugt wird. Das Kondensat des Stroms K wird dann vorzugsweise als Rücklauf zur Destillationskolonne DM zurückgeführt. Dazu kann, sofern notwendig, eine Pumpe eingesetzt werden.

Die Energieübertragung in Schritt c) von Strom K auf den Wärmeträger WA erfolgt in einem Verdampfer. Geeignete Verdampfer, die in Schritt k) eingesetzt werden können, sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Bevorzugt sind Kettleverdampfer. Neben den genannten kann aber auch jede beliebige andere, dem Fachmann bekannte Verdampferbauart, die sich zum Einsatz an einer Destillationskolonne eignet, eingesetzt werden.

In einer bevorzugten Ausführungsform umfasst der Verdampfer in Schritt c) zur Energieübertragung von Strom K auf den Wärmeträger WA strukturierte Wärmeübertragerrohre mit mindestens einem strukturierten Bereich. Strukturierte Wärmeübertragerrohre im Rahmen der vorliegenden Erfindung sind als Rohre zu verstehen, die mindestens einen strukturierten Bereich umfassen. Ein strukturierter Bereich zeichnet sich dabei durch ein wiederkehrendes Muster aus. Beispiele dafür sind Rippen bzw. Kanäle. Entsprechende Rohre sind dem Fachmann beispielsweise aus der EP 1 312 885 A2, der EP 1 830 151 A1, der WO 2017/207091 A1 oder der WO 2022/106045 A1 bekannt.

Die strukturierten Wärmeübertragerrohre weisen vorzugsweise mehr als einen, also mehrere strukturierte Bereiche auf. Sofern mehrere strukturierte Bereiche vorliegen, ist es bevorzugt, dass die strukturierten Wärmeübertragerrohre die strukturierten Bereiche auf der Rohrinnen- und/oder Rohraußenseite aufweisen. Bevorzugt ist sowohl auf der Rohrinnen- als auch der Rohraußenseite mindestens ein strukturierter Bereich vorhanden. In einer besonders bevorzugten Ausführungsform der vorliegenden Erfindung umfassen die strukturierten Bereiche kontinuierlich oder diskontinuierlich verlaufende achsparallele oder helixförmig umlaufende Rippen.

Der Einsatz strukturierter Wärmeübertragerrohre hat den Vorteil, dass weitere Verdichterleistung und damit elektrische Energie (Stromkosten) eingespart werden kann. Die strukturierten Bereiche bewirken einen verbesserten Wärmeaustausch zwischen den Medien in den Sumpfverdampfern.

Es versteht sich, dass die beiden Aspekte der vorliegenden Erfindung sowohl einzeln als auch in Kombination, d. h. die beanspruchte logarithmische Temperaturdifferenz und/oder der Einsatz strukturierter Wärmeübertragerrohre, vorliegen können.

WA kann ein beliebiger geeigneter Wärmeträger sein. Bevorzugt wird jedoch Wasser eingesetzt. Der Dampfstrom DS1 ist dann vorzugsweise ein Wasserdampfstrom. Der so erzeugte Dampfstrom DS1, vorzugsweise Wasserdampfstrom, weist nach der Energieübertragung in Schritt c) einen Druck im Bereich von 2 bis 5 bar absolut, vorzugsweise 2,2 bis 4 bar absolut auf.

### Schritt d)

In Schritt d) des erfindungsgemäßen Verfahrens erfolgt die Spaltungsreaktion von MTBE mit dem Strom K in der Gasphase. Die Spaltung wird in mindestens einem Reaktor unter Verwendung eines heterogenen Katalysators durchgeführt, wodurch ein Reaktionsgemisch R erhalten wird, das zumindest Isobuten, Methanol, Wasser und MTBE enthält, wobei der der Strom K vor dem Eintritt in den mindestens einen Reaktor und/oder der mindestens eine Reaktor mit einem Wärmeträgerfluid beheizt wird, das über einen Wärmeträgerfluidkreislauf bereitgestellt wird.

Bei der Spaltungsreaktion in Schritt d) können alle festen Katalysatoren eingesetzt werden, die im Temperaturbereich von 120 bis 400 °C, insbesondere im Bereich von 180 bis 350 °C, besonders bevorzugt im Bereich von 200 bis 250 °C, die Spaltung von MTBE zu Isobuten und Methanol bewirken. Die im erfindungsgemäßen Verfahren eingesetzten Katalysatoren können z. B. Metalloxide, Metallmischoxide, insbesondere solche, die Siliziumoxid und/oder Aluminiumoxid enthalten, Säuren auf Metalloxidträgern oder Metallsalze oder Mischungen davon sein. In Schritt d) des erfindungsgemäßen Verfahrens werden zur Spaltung von MTBE zu Isobuten und Methanol in der Gasphase bevorzugt Katalysatoren verwendet, die formal aus Magnesiumoxid, Aluminiumoxid und Siliziumoxid bestehen.

Besonders bevorzugt werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Das Alkalimetalloxid kann ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277:2010) von 200 bis 450 m²/g, bevorzugt von 200 bis 350 m²/g auf. Wird der erfindungsgemäße Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g. Der mittlere Porendurchmesser (vorzugsweise bestimmt in Anlehnung an DIN ISO 15901-1 von 2019) des Katalysators für die Spaltung in Schritt d) beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer gleich 3,5 nm bestimmt mit Quecksilberporosimetrie gemäß DIN 66133) des Katalysators auf Poren mit einem Durchmesser von 3,5 bis 50 nm (Mesoporen).

In Schritt d) des erfindungsgemäßen Verfahrens kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Es kann für die Aktivität und / oder Selektivität einiger Katalysatoren vorteilhaft sein, wenn dem Reaktorzulauf Anteile an Wasser zugegeben werden. Im erfindungsgemäßen Verfahren kann die Zugabe von Wasser zum Reaktorzulauf beispielsweise durch Zugabe von Wasser in den Strom K erfolgen. Dies erfolgt beispielsweise direkt durch Zugabe von Wasser oder Wasserdampf in den Strom K der dem mindestens einen Reaktor zugeleitet wird. Wird der Strom K vor dem Reaktor separat verdampft, kann hier Wasser zugegeben werden. Eine weitere Möglichkeit besteht darin, das Wasser schon im Verfahrensschritt c) bei der Abtrennung von MTBE zuzugeben. Bevorzugt wird dabei das Wasser in den Kolonnenzulauf oder in den Rücklauf der Kolonne DM eingespeist.

Die optionale Zugabe von Wasser zur Moderation des Katalysators erfolgt derart, dass der Wasseranteil in Strom K bevorzugt 0 bis 5 Massen-%, besonders bevorzugt 0,2 bis 1,5 Massen-% beträgt. Als zugeführtes Wasser wird dabei bevorzugt vollständig demineralisiertes oder destilliertes Wasser oder Wasserdampf eingesetzt.

Die Spaltung des MTBE in Schritt d) wird in der Gasphase vorzugsweise im Temperaturbereich von 120 bis 400 °C, insbesondere 180 bis 350 °C, besonders bevorzugt 200 bis 250 °C durchgeführt. Der Druck bei der Spaltung in Schritt d) liegt vorzugsweise im Bereich von 1 bis 20 bar absolut, weiterhin bevorzugt im Bereich von 0,3 bis 10 bar absolut, besonders bevorzugt im Bereich von 5 bis 8 bar absolut.

Die Spaltung von MTBE in Isobuten und Methanol ist eine endotherme Reaktion, dass bedeutet, dass sich die Reaktionsmischung bei der Reaktion abkühlt. Deswegen wird der Strom K vor dem Eintritt in den mindestens einen Reaktor und/oder der mindestens eine Reaktor mit einem Wärmeträgerfluid beheizt, das über einen Wärmeträgerfluidkreislauf bereitgestellt wird. So wird sichergestellt, dass der Strom K mit ausreichend hoher Temperatur in den Reaktor gelang. Geeignete Wärmeträgerfluide sind im Rahmen der vorliegenden Erfindung Marlotherm^{®}, Hexan und Wasser.

Bevorzugt wird der Reaktor derart betrieben, dass es unter den gewählten Druckbedingungen zu keiner Partialkondensation von MTBE und/oder Produkten am Katalysator kommt. Besonders bevorzugt wird der Reaktor so betrieben, dass die Minimaltemperatur im Reaktor an jeder Stelle des Katalysatorbetts größer als 150 °C, besonders bevorzugt größer 200 °C ist. Der maximale Temperaturabfall kann durch zahlreiche Parameter, wie z. B. durch die Temperatur des zum Heizen verwendeten Wärmeträgers sowie durch die Geschwindigkeit, mit der der Wärmträger durch den Mantel strömt, eingestellt werden. Bevorzugt wird das Temperaturprofil im Katalysatorbett durch eine ausreichende Anzahl von Temperaturmessungen überwacht.

Im Laufe des Betriebes kann es vorteilhaft sein, mit zunehmender Desaktivierung des Katalysators zur Konstanthaltung des Umsatzes die Eingangs- und / oder Betriebstemperatur anzuheben.

Der Umsatz des MTBEs in Schritt c) des erfindungsgemäßen Verfahrens beträgt zwischen 40 % und 98 %, bevorzugt zwischen 75 % und 97 %, besonders bevorzugt zwischen 85 % und 95 %.

Als Reaktoren werden bevorzugt Rohrreaktoren oder Rohrbündelreaktoren, insbesondere solche mit inneren Rohrdurchmessern von 10 bis 60 mm, verwendet. Neben Rohrbündelapparaten können auch Plattenreaktoren für die Durchführung der Spaltungsreaktion eingesetzt werden. Plattenreaktoren sind analog zu Plattenwärmetauschern aufgebaut. Der Abstand der Platten, zwischen denen sich der Katalysator befindet, beträgt dabei bevorzugt 10 bis 80 mm.

Bei der Spaltung von MTBE können Nebenreaktionen auftreten. Diese sind entweder auf MTBE oder die Spaltprodukte Isobuten und Methanol zurückzuführen, beispielsweise die Bildung von Dimethylether (DME) auf, die Bildung von C8-Kohlenwasserstoffen durch Dimerisierung von Isobuten, die Bildung von Isobutan, Isopren und Dimethoxymethan sowie höherer Oligomere (C12- oder C16-Kohlenwasserstoffe) oder die Bildung von Buten durch die Spaltung von 2-Methoxybutan.

Da die Spaltungsreaktion bei recht hoher Temperatur durchgeführt wird, kann das Reaktionsgemisch R benutzt werden, um andere Ströme innerhalb des Verfahrens vorzuwärmen. In einer bevorzugten Ausführungsform wir das Reaktionsgemisch R zur Vorwärmung des Stroms LS vor dem Zuführen in die Destillationskolonne DM in Schritt c) und/oder zur Vorwärmung des Stroms K vor dem Spaltungsreaktion in Schritt d) und/oder zur Vorwärmung des in die optional vorhandene Destillationskolonne DW2 geführten Stroms eingesetzt. Bevorzugt werden zwei oder alle drei Vorwärmungen realisiert.

Anschließend wird das Reaktionsgemisch R zumindest teilweise gemäß Schritt h) kondensiert, wobei die Kondensationswärme dadurch genutzt wird, dass Energie vom Reaktionsgemisch R auf einen flüssigen oder gasförmigen Wärmeträger W übertragen wird, wodurch ein Wärmeträger W2 entsteht.

Nach der Vorwärmung und der Energieübertragung gemäß Schritt h) kann das Reaktionsgemisch R zweiphasig vorliegen, weil sowohl eine Gasphase als auch aufgrund der Kondensation bei der Vorwärmung eine Flüssigphase vorliegt. Das Reaktionsgemisch R muss im nachfolgenden Schritt e) für die Destillation verdampft werden. Um Energie zu sparen, wird vorzugsweise vor dem Zuführen zur Destillationskolonne DR ein Flashbehälter vorgesehen, in den das Reaktionsgemisch R geflasht wird. Dadurch wird sich eine flüssige Phase ausbilden, die optional mit einer Pumpe in die Destillationskolonne DR geleitet wird. Die im Flashbehälter anfallende Gasphase kann optional über einen Kondensator gefahren werden, um einen Teil auszukondensieren und die Energie an anderer Stelle zu nutzen. Das anfallende Kondensat wird ebenfalls in den Flashbehälter geleitet. Die verbleibende Gasphase wird der Destillationskolonne DR zugeführt, wobei diese Phase räumlich gesehen oberhalb der Flüssigphase in die Destillationskolonne eingeleitet wird.

### Schritt e)

Der Schritt e) betrifft die Destillation des aus Schritt d) erhaltenen Reaktionsgemischs R in mindestens einer Destillationskolonne DR. Dabei fallen ein Strom IB, der zumindest Isobuten enthält, und ein Strom M, der zumindest Methanol und Wasser enthält, an. In einer bevorzugten Ausführungsform enthält der Strom M jeweils mehr als 50 Massen-% der in Reaktionsgemisch R enthaltenen Methanol- und Wasser-Menge.

In einer bevorzugten Ausführungsform erfolgt die destillative Auftrennung des Reaktionsgemischs R in Schritt e) in genau einer Destillationskolonne DR. Diese Destillationskolonne DR weist vorzugsweise von 20 bis 55 theoretische Trennstufen, weiterhin bevorzugt von 25 bis 45 theoretische Trennstufen und besonders bevorzugt von 30 bis 40 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne kann vorzugsweise zwischen 1 und 20 bar absolut eingestellt werden. Dabei ist es vorteilhaft, die Kolonne DR bei einem niedrigeren Druck als den Druck, mit dem der Spaltung im vorherigen Schritt betrieben wird, zu betreiben. Auf einen Verdichter zur Anhebung des Drucks oder eine vollständige Kondensation kann so verzichtet werden.

Die Spaltung in Schritt d) findet bevorzugt in der Gasphase statt. Deshalb liegt das Reaktionsgemisch R ebenfalls zunächst in der Gasphase vor. Es ist im Rahmen der vorliegenden Erfindung jedoch bevorzugt, dass das Reaktionsgemisch R erst nach partieller Kondensation in die Destillationskolonne überführt wird. Dabei werden vorzugsweise 30 bis 70 Massen-%, besonders bevorzugt 40 bis 70 Massen-% des Reaktionsgemisches R kondensiert. Der nicht kondensierte Teil des Reaktionsgemischs R wird vorzugsweise direkt, der kondensierte Teil des Reaktionsgemisches R falls notwendig nach Druckerhöhung durch eine Pumpe, in die Kolonne DR eingebracht. Die bei der Abkühlung und partiellen Kondensation freiwerdende Wärme kann dabei zur Wärmeintegration innerhalb des Prozesses oder mit anderen Prozessteilen genutzt werden. Diese Wärmeintegration kann nach bekannten Standards der Technik, beispielsweise über Sumpfverdampfer oder Vorwärmer bzw. Vorverdampfung im Kolonnenzulauf, erfolgen. Möglich wäre auch der Einsatz einer Wärmepumpe.

Die Einspeisung des gasförmigen Teils des Reaktionsgemisches R und des flüssigen Teils des Reaktionsgemisches R kann dabei an gleicher oder unterschiedlicher Stelle der Kolonne DR erfolgen. Bevorzugt erfolgt die Einspeisung des flüssigen Teils des Reaktionsgemisches R kann auf dem gleichen Boden oder ein bis fünf Böden unterhalb der Einspeisung des gasförmigen Teils des Reaktionsgemisches R.

Als Sumpfprodukt wird bei der Destillation in Schritt e) der Strom M erhalten, der vorzugsweise jeweils mehr als 50 Massen-% der in Reaktionsgemisch R enthaltenen Methanol- und Wasser Menge enthält. Bei Aufarbeitung entsprechend dieser Ausführungsform kann Strom M zudem noch MTBE enthalten. Bevorzugt weist Strom M jeweils mehr als 90 Massen-% der in Reaktionsgemisch R enthaltenen Methanol-Menge, mehr als 85 Massen-% der in Reaktionsgemisch R enthaltenen Wasser-Menge und mehr als 98 Massen-% der in Reaktionsgemisch R enthaltenen MTBE-Menge auf. Besonders bevorzugt enthält Strom M praktisch die Gesamtmenge der in Reaktionsgemisch R enthaltenen MTBE-, 2-Methoxybutan- und Diisobuten-Menge.

Am Kopf der Kolonne DR wird der Strom IB erhalten, der zumindest das Isobuten enthält. Zusätzlich dazu kann der Strom IB Methanol (aufgrund der Azeotropbildung mit C4 Kohlenwasserstoffen) und DME enthalten. Der Gehalt an Methanol im Destillat beträgt vorzugsweise maximal 2 bis 4 Massen-%. In Spuren können auch 1-Buten, 2-Butene, 1,3 Butadien und Mittelsieder wie Isopren und Dimethoxymethan enthalten sein. Bevorzugt liegt der Gehalt an Mittelsiedern in Summe unter 50 ppm, besonders bevorzugt liegt der Gehalt an Isopren unter 20 ppm, der an Dimethoxymethan unter 30 ppm. Der Strom IB kann dann weiteren Aufarbeitungsschritten zugeführt werden, um einen möglichst reinen Isobutenstrom zu erhalten. Eine solche weitergehende Aufarbeitung ist beispielsweise in der EP 2 448 893 A1 offenbart.

In einer weiteren bevorzugten Ausführungsform wird in Schritt e) eine zweite Destillationskolonne DR2 eingesetzt. Damit kann das Sumpfprodukt der Destillation, also Strom M, in einer zweiten Destillation weiter aufgereinigt werden. Bei der Auftrennung wird eine hauptsächlich MTBE- und Methanol-haltige Kopffraktion erhalten. Daneben wird eine im Wesentlichen MTBE-freie, Methanol-reiche Sumpffraktion erhalten, in der auch der größte Teil des Wassers enthalten ist.

Diese zweite Destillationskolonne DR2 weist vorzugsweise 20 bis 75 theoretische Trennstufen, bevorzugt 30 bis 65 und besonders bevorzugt 35 bis 50 auf. Es kann vorteilhaft sein, wenn die Kolonne in Abhängigkeit von der realisierten Stufenzahl und des im Spaltungsreaktor erzielten MTBE-Umsatzes, mit einem Rücklaufverhältnis, von kleiner 10, bevorzugt von 0,5 bis 5 betrieben wird. Der Betriebsdruck der Kolonne wird vorzugsweise auf einen Wert im Bereich von 0,5 bis 10 bar absolut, bevorzugt von 1 bis 3 bar absolut eingestellt. Die Kondensation kann, je nach gewähltem Betriebsdruck, gegen Kühlsohle, Kühlwasser oder Luft erfolgen.

Die hauptsächlich MTBE und Methanol-haltige Kopffraktion enthält neben dem Hauptbestandteil MTBE noch Methanol und ggf. die im Reaktionsteil entstandenen Mittelsieder, beispielsweise Isopren und/oder Dimethoxymethan. Dieser Strom wird bevorzugt ganz oder teilweise in Schritt c) des erfindungsgemäßen Verfahrens geführt.

### Schritt f)

In Schritt f) folgt die Wasserabtrennung aus dem Strom M in einer einzigen Destillationskolonne DW1, wobei am Kopf der Destillationskolonne DW1 ein Strom WK, der zumindest Methanol enthält, und am Sumpf ein Strom, der zumindest Wasser enthält, anfällt, wobei der Strom WK einer ein- oder mehrstufigen Brüdenverdichtung unterworfen und dann zur Beheizung der Destillationskolonne DW1 eingesetzt wird.

Die Destillationskolonne DW1 weist vorzugsweise 20 bis 80 theoretische Trennstufen, bevorzugt 30 bis 70 und besonders bevorzugt 50 bis 65 auf. Es kann vorteilhaft sein, wenn die Kolonne in Abhängigkeit von der realisierten Stufenzahl und des im Spaltungsreaktor erzielten MTBE-Umsatzes, mit einem Rücklaufverhältnis, von kleiner 2, bevorzugt von 0,5 bis 1 betrieben wird. Der Betriebsdruck der Kolonne wird vorzugsweise auf einen Wert im Bereich von 0,5 bis 10 bar absolut, bevorzugt von 1 bis 5 bar absolut eingestellt. Zur Beheizung der Kolonne kann z. B. 4 bar Dampf eingesetzt werden. Die Kondensation kann, je nach gewähltem Betriebsdruck, gegen Kühlsohle, Kühlwasser oder Luft erfolgen.

Bei der Ausführung der Wasserabtrennung als eine Destillationskolonne wird der Destillatstrom WK erhalten. Hauptbestandteil des Stroms WK ist Methanol. Es kann auch MTBE enthalten sein. Der Wassergehalt im Strom WK beträgt weniger als 1 Massen-%, bevorzugt weniger als 0,5 Massen-%, besonders bevorzugt weniger als 0,1 Massen-% bezogen auf die gesamte Masse des Stroms WK. Strom WK kann auch vollständig oder teilweise zur MTBE-Synthese zurückgeführt werden.

Der Sumpfstrom der Kolonne DW1 besteht zum überwiegenden Teil aus Wasser. Der Strom kann Spuren von Methanol und gegebenenfalls von weiteren organischen Komponenten enthalten. Vorzugsweise ist der Wassergehalt größer als 99,9 Massen-%, bevorzugt größer als 99,99 Massen-% und besonders bevorzugt größer 99,999 Massen-%. Dieser Strom kann entweder als Prozesswasser genutzt werden, z. B. in einem Extraktionsschritt, oder als Abwasser, gegebenenfalls nach weiterer Reinigung, ausgeschleust werden.

In einer bevorzugten Ausführungsform der vorliegenden Erfindung weist die Destillationskolonne DW1 einen Seitenverdampfer STV auf. Grundsätzlich könnte der Seitenverdampfer STV auch den Sumpfverdampfer SV ersetzen, d. h. die Destillationskolonne DW1 könnte auch ausschließlich durch den Seitenverdampfer STV beheizt werden. Es ist erfindungsgemäße bevorzugt, wenn die Destillationskolonne DW1 einen Seitenverdampfer STV und einen Sumpfverdampfer SV aufweist.

Der Strom WK wird erfindungsgemäß einer ein- oder mehrstufigen Brüdenverdichtung unterworfen und dann zur Beheizung der Destillationskolonne DW1 eingesetzt wird. Die Beheizung erfolgt in der Regel über den Sumpfverdampfer SV. Für den Fall, dass ein Seitenverdampfer vorliegt, kann die Beheizung über den Sumpfverdampfer SV und/oder den Seitenverdampfer STV erfolgen.

Das Verdichten des Stroms WK kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck WK verdichtet werden soll.

Als Verdichter im erfindungsgemäßen Verfahren, insbesondere zum Verdichten des Stroms WK, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

In einer bevorzugten Ausführungsform der vorliegenden Erfindung erfolgt das Verdichten des Stroms WK mehrstufig. In einer besonders bevorzugten Ausführungsform wird der Strom WK in zwei Verdichterstufen verdichtet wird, wobei in der ersten Verdichtung ein verdichteter Wärmeträger WK1 entsteht, der einen höheren Druck als WK aufweist und in der zweiten Verdichtung der verdichtete Wärmeträger WK2 entsteht, der einen höheren Druck als WK1 und als WK aufweist. Die zweistufige Verdichtung findet vorzugsweise in einer einzigen Verdichtermaschine statt. Die Verdichtermaschine, mit der die mehrstufige Verdichtung durchgeführt wird, kann weiterhin bevorzugt einen oder mehrere Seitenabzüge aufweisen, wo Ströme unterschiedlicher Druckstufen entnommen werden können. Die Verdichtermaschine weist vorzugsweise maximal 7 Seitenabzüge auf.

In einer bevorzugten Ausführungsform der vorliegenden Erfindung wird der Strom WK verdichtet, wodurch der Strom WK1 entsteht, der anschließend zur Beheizung im Seitenverdampfer STV eingesetzt wird. Die Verdichtung kann ein- oder mehrstufig erfolgen, vorzugsweise erfolgt die Verdichtung zu WK1 aber mehrstufig, vorzugsweise in einer einzigen Verdichtermaschine. Geeignete Verdichter sind in den vorherigen Absätzen offenbart.

Es ist weiterhin bevorzugt, dass der Strom WK1 nach der Beheizung im Seitenverdampfer STV zu einem Flashbehälter geführt wird, wobei der Strom WK1 vor dem Eintritt in den Flashbehälter vorzugsweise zur Vorwärmung des Stroms WK vor der Verdichtung eingesetzt wird. Durch die Vorwärmung wird der Strom WK überhitzt. Dadurch wird die Bildung von Tröpfchen vor oder im Verdichter sicher verhindert, die sonst zu Schäden am Verdichter führen können.

Im Flashbehälter werden vorzugsweise eine gasförmige Phase, die mit dem Strom WK vor der Vorwärmung vermischt wird, und eine flüssige Phase anfallen, die zumindest teilweise zurück zur Destillationskolonne DW1 und teilweise unter Erhalt des Stroms WK2 verdichtet und anschließend zur Beheizung im Sumpfverdampfer SV eingesetzt wird. Optional wird die flüssige Phase zusätzlich teilweise mit dem Strom WK1 vor der Beheizung des Seitenverdampfers vermischt.

Die Verdichtung zu WK2 kann ein- oder mehrstufig erfolgen, vorzugsweise erfolgt die Verdichtung zu WK2 aber einstufig. Geeignete Verdichter sind in den vorherigen Absätzen offenbart.

Der Strom WK2 wird nach der Beheizung des Sumpfverdampfers vorzugsweise ebenfalls zum Flashbehälter geführt wird.

Das Zurückführen der flüssigen Phase zur Destillationskolonne DW1 und/oder das Zuführen der flüssigen Phase zur Verdichtung Zu WK2 mittels einer Pumpe erfolgt.

### Schritt g)

Schritt g) betrifft einen der wärmeintegrativen Schritte. Damit versteht sich, dass die Bezeichnung der Schritte keine chronologische Abfolge der Schritte darstellt, sondern die Schritte teils auch gleichzeitig oder in einer anderen Reihenfolge erfolgen können.

Schritt g) meint das zumindest teilweise Kondensieren des Kopfstroms LK, bei dem die Kondensationswärme dadurch genutzt wird, dass Energie vom Kopfstrom LK auf einen flüssigen oder gasförmigen Wärmeträger W übertragen wird, wodurch ein Wärmeträger W1 entsteht. Durch die Übertragung von Energie auf den Wärmeträger W wird sich der Energiegehalt des Stroms LK verringern, der Strom sich also abkühlen und zumindest teilweise kondensieren.

Als Wärmeträger W kann jedes dem Fachmann geläufige Arbeitsmedium genutzt werden. Der Wärmeträger W wird vorzugsweise aus der Gruppe bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt wird, vorzugsweise Wasser, Methanol, Ethanol, Propanol, n-Pentan, n-Butan, n-Hexan, n-Propan oder Ammoniak, besonders bevorzugt n-Pentan ist.

Wird der Wärmeträger W in Schritt g) in flüssiger Phase, also ein flüssiger Wärmeträger W, eingesetzt und diesem in Schritt g) Energie, bevorzugt Wärme, zugeführt, wird der Wärmeträger W zumindest teilweise verdampft und dadurch ein gasförmiger Wärmeträger W1 erhalten. Wird der Wärmeträger W in Schritt g) in gasförmiger Phase, also ein gasförmiger Wärmeträger W eingesetzt und diesem in Schritt g) Energie, bevorzugt Wärme, zugeführt, wird ebenfalls ein gasförmiger Wärmeträger W1 erhalten.

Mit der Formulierung "flüssiger Wärmeträger" ist im Rahmen der vorliegenden Erfindung gemeint, dass > 50 Gew.-%, weiterhin bevorzugt > 55 Gew.-%, weiterhin bevorzugt > 75 Gew.-%, weiterhin bevorzugt > 90 Gew.-% und besonders bevorzugt > 99 Gew.-% des in Schritt g) eingesetzten Wärmeträgers im flüssigen Aggregatzustand vorliegen, jeweils bezogen auf das Gesamtgewicht des in Schritt g) eingesetzten Wärmeträgers.

Mit der Formulierung "gasförmiger Wärmeträger" ist im Rahmen der vorliegenden Erfindung gemeint, dass > 30 Gew.-%, weiterhin bevorzugt > 50 Gew.-%, weiterhin bevorzugt > 75 Gew.-%, weiterhin bevorzugt > 90 Gew.-% und besonders bevorzugt > 99 Gew.-% des in Schritt g) eingesetzten Wärmeträgers im gasförmigen Aggregatzustand vorliegen, jeweils bezogen auf das Gesamtgewicht des in Schritt g) eingesetzten Wärmeträgers.

Die Übertragung von Energie in Schritt g) kann nach dem Fachmann bekannten Verfahren oder mit dem Fachmann bekannten Wärmetauschern durchgeführt werden. Der Wärmetauscher ist in diesem Fall der Kondensator zur Kondensation des Stroms LK. Für die Übertragung von Energie in Schritt g) eignen sich insbesondere Kettle-Type-Wärmeübertrager.

Nach der beschriebenen Energieübertragung in Schritt g) weist der Wärmeträger W1 gegenüber dem Wärmeträger W vorzugsweise eine erhöhte Temperatur und/oder einen erhöhten Druck auf. In einer bevorzugten Ausführungsform der vorliegenden Erfindung weist W1 eine Temperatur im Bereich von 30 °C bis 80 °C auf. Der Druck von W1 liegt vorzugsweise im Bereich von 1 bar bis 20 bar, bevorzugt 1,5 bar bis 5 bar auf.

Es versteht sich von selbst, dass der Wärmeträger W1 dem Wärmeträger W entspricht und sich W und W1 nur in ihrem jeweiligen Druck und/oder ihrer Temperatur und gegebenenfalls, wenn W als Flüssigkeit eingesetzt wurde, durch den Aggregatzustand unterscheiden.

Der durch die Wärmeübertragung zumindest teilweise kondensierte Strom LK kann teilweise zurück zur Destillationskolonne DL geführt werden. Ein anderer Teil des in Schritt g) kondensierten Kopfstroms LK kann aus dem Verfahren ausgeschleust werden.

### Schritt h)

Schritt h) betrifft einen weiteren der wärmeintegrativen Schritte. Damit versteht sich, dass die Bezeichnung der Schritte keine chronologische Abfolge der Schritte darstellt, sondern die Schritte teils auch gleichzeitig oder in einer anderen Reihenfolge erfolgen können.

Schritt h) meint das zumindest teilweise Kondensieren des Reaktionsgemisches R, bei dem die Kondensationswärme dadurch genutzt wird, dass Energie vom Reaktionsgemisch R auf einen flüssigen oder gasförmigen Wärmeträger W übertragen wird, wodurch ein Wärmeträger W2 entsteht. Durch die Übertragung von Energie auf den Wärmeträger W wird sich der Energiegehalt des Reaktionsgemisches R verringern, der Strom sich also abkühlen und zumindest teilweise kondensieren.

Der Wärmeträger W aus Schritt h) ist der gleiche Wärmeträger, der in Schritt g) eingesetzt wird. Als Wärmeträger W kann jedes dem Fachmann geläufige Arbeitsmedium genutzt werden. Der Wärmeträger W wird vorzugsweise aus der Gruppe bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt, weiterhin bevorzugt Wasser, Methanol, Ethanol, Propanol, n-Pentan, n-Butan, n-Hexan, n-Propan oder Ammoniak, besonders bevorzugt n-Pentan ist.

Wird der Wärmeträger W in Schritt h) in flüssiger Phase, also ein flüssiger Wärmeträger W, eingesetzt und diesem in Schritt h) Energie, bevorzugt Wärme, zugeführt, wird der Wärmeträger W zumindest teilweise verdampft und dadurch ein gasförmiger Wärmeträger W2 erhalten. Wird der Wärmeträger W in Schritt h) in gasförmiger Phase, also ein gasförmiger Wärmeträger W eingesetzt und diesem in Schritt h) Energie, bevorzugt Wärme, zugeführt, wird ebenfalls ein gasförmiger Wärmeträger W2 erhalten.

Mit der Formulierung "flüssiger Wärmeträger" ist im Rahmen der vorliegenden Erfindung gemeint, dass > 50 Gew.-%, weiterhin bevorzugt > 55 Gew.-%, weiterhin bevorzugt > 75 Gew.-%, weiterhin bevorzugt > 90 Gew.-% und besonders bevorzugt > 99 Gew.-% des in Schritt h) eingesetzten Wärmeträgers im flüssigen Aggregatzustand vorliegen, jeweils bezogen auf das Gesamtgewicht des in Schritt h) eingesetzten Wärmeträgers.

Mit der Formulierung "gasförmiger Wärmeträger" ist im Rahmen der vorliegenden Erfindung gemeint, dass > 30 Gew.-%, weiterhin bevorzugt > 50 Gew.-%, weiterhin bevorzugt > 75 Gew.-%, weiterhin bevorzugt > 90 Gew.-% und besonders bevorzugt > 99 Gew.-% des in Schritt h) eingesetzten Wärmeträgers im gasförmigen Aggregatzustand vorliegen, jeweils bezogen auf das Gesamtgewicht des in Schritt h) eingesetzten Wärmeträgers.

Die Übertragung von Energie in Schritt h) kann nach dem Fachmann bekannten Verfahren oder mit dem Fachmann bekannten Wärmetauschern durchgeführt werden. Der Wärmetauscher ist in diesem Fall der Kondensator zur Kondensation des Reaktionsgemisches R. Für die Übertragung von Energie in Schritt h) eignen sich insbesondere Kettle-Type-Wärmeübertrager.

Nach der beschriebenen Energieübertragung in Schritt g) weist der Wärmeträger W2 gegenüber dem Wärmeträger W vorzugsweise eine erhöhte Temperatur und/oder einen erhöhten Druck auf. In einer bevorzugten Ausführungsform der vorliegenden Erfindung weist W2 eine Temperatur im Bereich von 30 °C bis 80 °C auf. Der Druck von W1 liegt vorzugsweise im Bereich von 1 bar bis 20 bar, bevorzugt 1,5 bis 5 bar auf.

Es versteht sich von selbst, dass der Wärmeträger W2 dem Wärmeträger W entspricht und sich W und W2 nur in ihrem jeweiligen Druck und/oder ihrer Temperatur und gegebenenfalls, wenn W als Flüssigkeit eingesetzt wurde, durch den Aggregatzustand unterscheiden.

Das durch die Wärmeübertragung zumindest teilweise kondensierte Reaktionsgemisch R kann teilweise

### Schritt i)

In Schritt i) des erfindungsgemäßen Verfahrens werden die beiden Wärmeträger W1 und W2 vermischt und es entsteht der Wärmeträger W3. Die Vermischung kann ohne besondere Einbauten einfach durch das Zusammenführen der beiden Rohrleitungen erfolgen. Dies ist dem Fachmann grundsätzlich geläufig. Die Vermischung erfolgt dadurch, dass die beiden Leitungen, beispielsweise Rohrleitungen zusammengeführt werden. Durch die Verdichtung im nachfolgenden Schritt i) werden beide Wärmeträger W1 und W2 angesaugt und vermischt.

### Schritt j)

Im nachfolgenden Schritt j) wird zumindest ein Teil des aus Schritt i) erhaltene Wärmeträgers W3, vorzugsweise der gesamte Wärmeträger W3 verdichtet, wodurch ein verdichteter Wärmeträger W4 entsteht, der einen höheren Druck aufweist als der Wärmeträger W3. Der Druck von W4 liegt nach dem Verdichten vorzugsweise im Bereich von 1 bar bis 20 bar, bevorzugt 5 bar bis 15 bar auf.

Das Verdichten des mindestens einen Teils des Wärmeträgers W3 in Schritt j) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Wärmeträger W3 verdichtet werden soll.

Als Verdichter im erfindungsgemäßen Verfahren, insbesondere zum Verdichten des Wärmeträgers W3, eignet sich jeder beliebige, dem Fachmann bekannte Verdichter, bevorzugt mechanische Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

In einer bevorzugten Ausführungsform der vorliegenden Erfindung erfolgt das Verdichten des Wärmeträgers W3 mehrstufig. In einer besonders bevorzugten Ausführungsform wird der Wärmeträger W3 in zwei Verdichterstufen V1 und V2 verdichtet wird, wobei in der ersten Verdichtung ein verdichteter Wärmeträger W3.1 entsteht, der einen höheren Druck als W3 aufweist und in der zweiten Verdichtung der verdichtete Wärmeträger W4 entsteht, der einen höheren Druck als W3.1 und als W3 aufweist. Die zweistufige Verdichtung findet vorzugsweise in einer einzigen Verdichtermaschine statt. Auch diese Verdichtermaschine kann einen oder mehrere Seitenabzüge aufweisen.

### Schritt k)

Der sich anschließende Schritt k) betrifft dann die Wärmeübertragung. Dabei wird Energie vom verdichteter Wärmeträger W4 auf einen weiteren Wärmeträger WB übertragen, wodurch ein Dampfstrom DS2 erzeugt wird, der teilweise zur Beheizung der Destillationskolonne DL, der Destillationskolonne DR und optional der Destillationskolonne DW1 und teilweise mit dem Dampfstrom DS1 vermischt, unter Erhalt eines verdichteten Dampfstroms DS3 verdichtet und anschließend zur Beheizung der Destillationskolonne DM und nach weiterer Verdichtung unter Erhalt eines weiter verdichteten Dampfstroms DS4 zur Beheizung des Wärmeträgerfluidkreislaufs eingesetzt wird.

Die Beheizung der Destillationskolonne DW1 ist optional, weil hier eine Wärmeintegration mit einer möglichen zweiten Destillationskolonne DW2 mittels einer Zweidruckschaltung oder einer Brüdenverdichtung stattfinden kann (s. Fig. 4). Es ist jedoch auch möglich die Destillationskolonne DW1 mit dem Dampfstrom DS2 zu beheizen (s. Fig. 3).

Schritt k) sieht also vor, dass zunächst Energie auf einen weiteren Wärmeträger WB übertragen wird. Die Übertragung von Energie in Schritt k) erfolgt dann so, dass über den verdichteten Wärmeträger W4 in einem Verdampfer Dampf (Dampfstrom DS2) erzeugt wird, der zur Beheizung eingesetzt wird. Der Dampf ist vorliegend vorzugsweise Wasserdampf. Der in Schritt k) erzeugte Dampfstrom DS2 ist somit vorzugsweise ein Wasserdampfstrom. Da die beiden Ströme DS1 und DS2 vermischt werden, ist es von Vorteil, wenn beide Ströme identisch sind. DS1 ist deshalb vorzugsweise ebenfalls ein Wasserdampfstrom. Die Wärmeträger WA und WB sind demnach bevorzugt Wasser.

Die Energieübertragung in Schritt k) von W4 auf den weiteren Wärmeträger WB erfolgt in einem Verdampfer. Geeignete Verdampfer, die in Schritt k) eingesetzt werden können, sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Bevorzugt sind Kettleverdampfer. Neben den genannten kann aber auch jede beliebige andere, dem Fachmann bekannte Verdampferbauart, die sich zum Einsatz an einer Destillationskolonne eignet, eingesetzt werden.

In einer bevorzugten Ausführungsform umfasst der Verdampfer in Schritt k) strukturierte Wärmeübertragerrohre mit mindestens einem strukturierten Bereich. Strukturierte Wärmeübertragerrohre im Rahmen der vorliegenden Erfindung sind als Rohre zu verstehen, die mindestens einen strukturierten Bereich umfassen. Ein strukturierter Bereich zeichnet sich dabei durch ein wiederkehrendes Muster aus. Beispiele dafür sind Rippen bzw. Kanäle. Entsprechende Rohre sind dem Fachmann beispielsweise aus der EP 1 312 885 A2, der EP 1 830 151 A1, der WO 2017/207091 A1 oder der WO 2022/106045 A1 bekannt.

Die strukturierten Wärmeübertragerrohre weisen vorzugsweise mehr als einen, also mehrere strukturierte Bereiche auf. Sofern mehrere strukturierte Bereiche vorliegen, ist es bevorzugt, dass die strukturierten Wärmeübertragerrohre die strukturierten Bereiche auf der Rohrinnen- und/oder Rohraußenseite aufweisen. Bevorzugt ist sowohl auf der Rohrinnen- als auch der Rohraußenseite mindestens ein strukturierter Bereich vorhanden. In einer besonders bevorzugten Ausführungsform der vorliegenden Erfindung umfassen die strukturierten Bereiche kontinuierlich oder diskontinuierlich verlaufende achsparallele oder helixförmig umlaufende Rippen.

Der Einsatz strukturierter Wärmeübertragerrohre hat den Vorteil, dass weitere Verdichterleistung und damit elektrische Energie (Stromkosten) eingespart werden kann. Die strukturierten Bereiche bewirken einen verbesserten Wärmeaustausch zwischen den Medien in den Sumpfverdampfern.

Es versteht sich, dass die beiden Aspekte der vorliegenden Erfindung sowohl einzeln als auch in Kombination, d. h. die beanspruchte logarithmische Temperaturdifferenz und/oder der Einsatz strukturierter Wärmeübertragerrohre, vorliegen können.

Der so erzeugte Dampf, vorzugsweise Wasserdampf, also der Dampfstrom DS2 weist nach der Energieübertragung in Schritt k) einen Druck im Bereich von 2 bis 5 bar absolut, vorzugsweise 2,2 bis 4 bar absolut auf.

Mit einem Teil des so erzeugten Dampfstroms DS2, vorzugsweise einem Teil des Wasserdampfes, werden die Destillationskolonne DL, die Destillationskolonne DR und die Destillationskolonne DW1 beheizt, um die gewünschte Trennaufgabe bewältigen zu können. Sofern bei der Wasserabtrennung eine zweite Destillationskolonne DW2 vorliegt, kann der Dampfstrom DS2 auch zur Beheizung dieser Kolonne eingesetzt werden. Die Destillationskolonnen werden vorzugsweise über sogenannte Sumpfverdampfer beheizt, bei denen ein Teil des Sumpfs durch den Sumpfverdampfer geführt, erhitzt und dann wieder in die Destillationskolonne zurückgeführt wird.

Geeignete Verdampfer, die als Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Bevorzugt sind Naturumlaufverdampfer und Kettleverdampfer. Als Wärmeübertrager für den Verdampfer wird bei Naturumlaufverdampfern und Zwangsumlaufverdampfern üblicherweise ein Rohrbündel oder Plattenapparat eingesetzt. Neben den genannten kann aber auch jede beliebige andere, dem Fachmann bekannte Verdampferbauart, die sich zum Einsatz an einer Desorptionskolonne eignet, eingesetzt werden. Auch in diesem Fall kann Sumpfverdampfer strukturierte Wärmeübertragerrohre mit mindestens einem strukturierten Bereich, wie sie weiter oben beschrieben wurden, umfassen.

Durch das Beheizen der Destillationskolonne DL, der Destillationskolonne DR und der Destillationskolonne DW1 wird zumindest ein Teil des Dampfstroms DS2 kondensieren und als Kondensat anfallen. Das Kondensat wird dann als WB zur Wärmeübertragung zurückgeführt und durch die Wärmeübertragung von W4 erneut verdampft.

Der andere Teil des so erzeugten Dampfstroms DS2 wird zunächst mit dem Dampfstrom DS1 aus Schritt c) vermischt, anschließend verdichtet und dann nach Erhalt des verdichteten Dampfstroms DS3 zur Beheizung der Destillationskolonne DM eingesetzt. Bei der Beheizung wird ein Teil des Dampfstroms DS3 kondensieren. Das Kondensat wird dann ebenfalls als WB zur Wärmeübertragung zurückgeführt und durch die Wärmeübertragung von W4 erneut verdampft. Der verdichtete Dampfstrom DS3 weist gegenüber dem Dampfstrom DS2 eine erhöhte Temperatur und/oder einen erhöhten Druck auf. Der Dampfstrom DS3 weist nach der Verdichtung vorzugsweise einen Druck im Bereich von 8 bis 20 bar absolut, besonders bevorzugt 10 bis 18 bar absolut auf und wird anschließend zur Beheizung der Destillationskolonne DM eingesetzt.

Das Verdichten des anderen Teils des so erzeugten Dampfstroms DS2, der mit dem Dampfstrom DS1 aus Schritt c) vermischt worden ist, kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck verdichtet werden soll.

Als Verdichter im erfindungsgemäßen Verfahren, insbesondere zum Verdichten des Dampfstroms DS2, eignet sich jeder beliebige, dem Fachmann bekannte Verdichter, bevorzugt mechanische Verdichter, mit dem sich auch Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Der nach der Beheizung der Destillationskolonne DM noch vorhandene restliche Dampfstrom DS3 wird anschließend erneut verdichtet, wodurch der noch weiter verdichtete Dampfstrom DS4 entsteht. Der weiter verdichtete Dampfstrom DS4 weist gegenüber dem Dampfstrom DS3 eine erhöhte Temperatur und/oder einen erhöhten Druck auf. Der Dampfstrom DS4 weist nach der Verdichtung vorzugsweise einen Druck im Bereich von 18 bis 66 bar absolut, besonders bevorzugt 21 bis 45 bar absolut auf und wird anschließend zur Beheizung des Wärmeträgerfluidkreislaufs eingesetzt.

Das Verdichten des Dampfstroms DS3 kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck verdichtet werden soll.

Als Verdichter im erfindungsgemäßen Verfahren, insbesondere zum Verdichten des Dampfstroms DS3, eignet sich jeder beliebige, dem Fachmann bekannte Verdichter, bevorzugt mechanische Verdichter, mit dem sich auch Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

Der Wärmeträger W4 wird bei der Energieübertragung in Schritt k) vorzugsweise zumindest teilweise kondensiert. Es ist für den Fall, dass der Wärmeträger W3 in den zwei Stufen V1 und V2 verdichtet wird, weiterhin bevorzugt, dass der zumindest teilweise kondensierte Wärmeträger W4 in einen Flashbehälter gefahren wird, der auf dem Druckniveau betrieben wird, das zwischen den beiden Verdichtungsstufen V1 und V2 liegt, wodurch in dem Flashbehälter eine gasförmige Phase und eine flüssige Phase anfallen.

In einer bevorzugten Ausführungsform wird der Flashanteil im Flashbehälter als Gasphase zwischen die beiden Verdichtungsstufen geleitet. Dadurch kann ein Teil der nach der Energieübertragung in Schritt k) im Wärmeträger W4 vorhandenen Energie genutzt werden. Der Flashanteil wird mit dem verdichteten Wärmeträger W3.1 vermischt, der zweiten Verdichtung V2 unterworfen und zur Energieübertragung in Schritt 3) geführt. Nur der flüssige Anteil im Flashbehälter wird dann vorzugsweise als Wärmeträger W zu Schritt g) und/oder zu Schritt h) geleitet und durchläuft die dann die weiteren Schritte. Der flüssige Anteil kann weiterhin zur Vorwärmung des Wärmeträgers W3 vor der Verdichtung V1 eingesetzt wird, wobei Energie vom flüssigen Anteil auf den Wärmeträger W3 übertragen wird. Der Wärmeträger W3 wird dadurch überhitzt und eine Kondensation bei der Verdichtung verhindert.

Die vorliegende Erfindung wird anhand der nachfolgenden Abbildungen näher erläutert. Die Abbildungen zeigen spezielle Ausführungsformen, die den Gegenstand der vorliegenden Erfindung nicht einschränken.

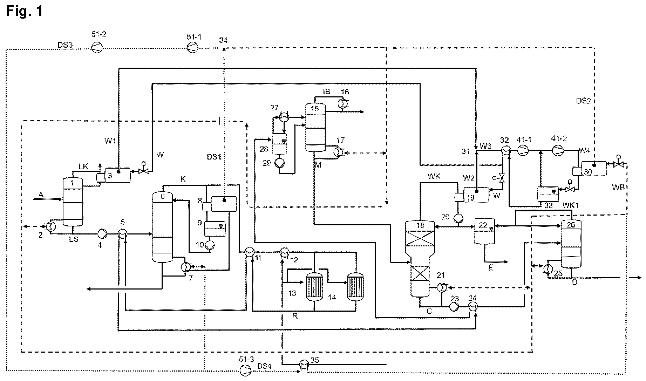

Fig. 1 ist eine schematische Darstellung einer MTBE-Spaltung und zeigt eine nicht erfindungsgemäße Ausgestaltung des Verfahrens. Ein Feedstrom (A), der MTBE enthält, wird dabei zu einer Leichtsiederabtrennung in der Leichtsiederkolonne DL (1) geführt. Die Leichtsiederkolonne (1) wird über einen Verdampfer (2) beheizt. Das Destillat LK, das am Kopf der Leichtsiederkolonne (1) anfällt, wird mithilfe eines Wärmeübertragers (3) gekühlt und teilweise ausgeschleust und teilweise als Rücklauf zu DL (1) zurückgeführt. Der Sumpfstrom LS enthält das MTBE und wird teilweise über eine Pumpe (4) und den Vorwärmer (5) zur Destillationskolonne DM (6) geleitet. Ein anderer Teil von LS wird über den Verdampfer (2) erhitzt und in den Sumpf der DL (1) zurückgeführt. Die Destillationskolonne DM (6) wird über den Verdampfer (7) beheizt. Hier findet hauptsächlich die Abtrennung des MTBE vom MSBE statt.

Am Kopf der Destillationskolonne DM (6) fällt der Strom K an, der teilweise im Wärmeübertrager (8) gekühlt wird. Am Wärmeübertrager (8) wird ein Heizdampf erzeugt, mit dem die Verdampfer (2), (17) und (25) beheizt werden. Ein anderer Teil des Heizdampfes kann ausgeschleust und an anderer Stelle eingesetzt werden, was durch den nach oben gerichteten Pfeil (b) angedeutet wird. Die flüssige Phase geht in den Phasentrenner (9) und über die Pumpe (10) als Rücklauf zur Destillationskolonne DM (6). Ein anderer Teil des Stroms K wird über die beiden Vorwärmer (11, 12) zu den beiden Spaltungsreaktoren (13, 14) geführt. Der Vorwärmer (12) wird mit dem Wärmeträgerfluid aus dem Wärmeträgerfluidkreislauf gespeist, was in der Zeichnung nur angedeutet wird. Dort wird MTBE in Isobuten und Methanol gespalten. Das erhaltene Reaktionsgemisch R wird genutzt, um im Vorwärmer (11) Energie auf den Strom K, im Vorwärmer (5) Energie auf den Strom LS und im Vorwärmer (24) auf den Sumpfstrom der DW1 (D) zu übertragen. Anschließend wird das Reaktionsgemisch R zur Destillationskolonne DR (15) geführt, die über den Verdampfer (17) beheizt wird. Bevor Strom R in die Destillationskolonne DR (15) gelangt, wird er erst in einen Phasentrennbehälter (28) geleitet und in eine wässrige und eine gasförmige Phase getrennt wird. Die wässrige Phase wird am unteren Ende des Phasentrennbehälters (28) abgenommen und mit einer Pumpe in die DR (15) geführt. Die gasförmige Phase wird über einen Kondensator (27) zur DR (15) geführt, wobei die Zuleitung der gasförmigen Phase oberhalb der Zuleitung der wässrigen Phase erfolgt. Am Kondensator (27) wird ein Teil auskondensieren und zum Phasentrennbehälter (28) geleitet

In der Destillationskolonne DR fällt am Kopf der Strom IB an, der zumindest Isobuten enthält. IB wird über den Wärmetauscher (16) gekühlt, teilweise als Rücklauf zur Kolonne DR (15) geführt und teilweise der weiteren Aufarbeitung zugeführt (nicht in Fig. 1 gezeigt). Am Sumpf der DR (15) fällt der Strom M an, der zumindest teilweise der Wasserabtrennung in der Destillationskolonne DW1 (18) und der Destillationskolonne DW2 (26) zugeführt wird. Die DW1 wird über den Sumpfverdampfer (21) beheizt, der mit dem Kopfstrom WK der Destillationskolonne DW2 (26) wärmintegriert ist. Am Kopf der Kolonne DW1 fällt ein Methanol-haltiger Strom WK an, der über den Wärmetauscher (19) gekühlt und mittels einer Pumpe (20) teilweise als Rücklauf zur DW1 (18) und teilweise zum Behälter (22) geleitet wird. Der Sumpfstrom (C), der hauptsächlich Wasser enthält, wird zumindest teilweise über eine Pumpe (23) und den Vorwärmer (24) zur Destillationskolonne DW2 (26) geführt. Dort fällt am Sumpf ein Wasserhaltiger Strom (D) an. Am Kopf der DW2 (26) fällt der Strom WK1 an, der nach Wärmeintegration mit dem Verdampfer (21) ebenfalls zum Behälter (22) geführt, wo das Methanol (E) abgenommen wird.

Am Wärmeüberträger (3), vorzugsweise einem Kettleverdampfer, wird Energie vom Kopfstrom LK auf einen Wärmeträger W übertragen und es entsteht ein Wärmeträger W1, der eine höhere Temperatur aufweist als W. Ebenso wird am Wärmeübertrager (19), vorzugsweise einem Kettleverdampfer, Energie vom Kopfstrom WK auf einen Wärmeträger W übertragen und es entsteht ein Wärmeträger W2, der eine höhere Temperatur aufweist als W. Die beiden Wärmeträger W1 und W2 werden anschließend vermischt (31) und es entsteht W3. W3 wird danach einer Verdichtung in den vorzugsweise zwei Verdichtern oder einem Verdichter mit zwei Stufen (41-1 und 41-2) unterworfen und es entsteht W4. Mit W4 wird Energie auf den weiteren Wärmeträger WB übertragen und es wird der Dampfstrom DS2 erzeugt. W4 kondensiert bei der Wärmeübertragung in (30) zumindest partiell und wird zum Phasentrennbehälter (33) geführt, wo eine gasförmige Phase, die zwischen die beiden Verdichter(stufen) (41-1 und 41-2) geführt wird, und eine flüssige Phase anfällt, die über den Vorwärmer als W zurück zum Wärmeübertrager (19) geführt wird. DS wird zur Beheizung der Verdampfer (2, 17, 21 und, sofern vorhanden, 25) eingesetzt. Ein weiterer Teil wird mit dem Strom DS1 aus dem Verdampfer (8) vermischt (34) und mit zwei Verdichtern oder einem Verdichter mit zwei Stufen (51-1 und 51-2) zum Dampfstrom DS3 verdichtet, der zur Beheizung des Verdampfers (7) eingesetzt wird. Ein weiterer Teil von DS3 wird im Verdichter (51-3) erneut verdichtet und zur Beheizung des Wärmeträgerfluidkreislaufs im Wärmeübertrager (35) eingesetzt. Anschließend wird das Kondensat als WB gemeinsam mit dem Kondensat von DS2 als WB zurück zum Wärmeübertrager (30) geleitet.

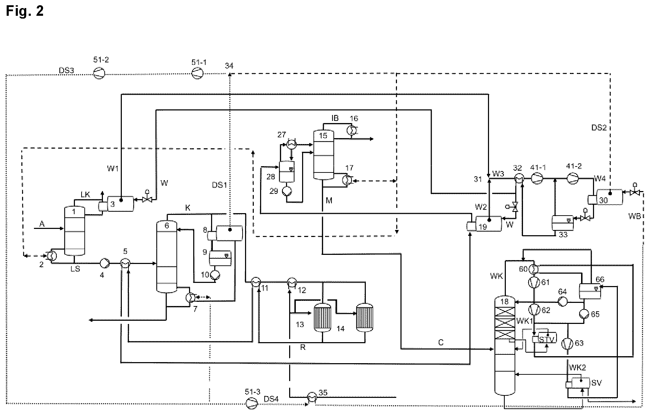

Fig. 2 zeigt eine erfindungsgemäße Ausführungsform, die in weiten Teilen den Ausführungsformen nach Fig. 1 entspricht. Der Unterschied besteht darin, dass die Wasserabtrennung in nur einer einzigen Destillationskolonne DW1 (18) stattfindet. Am Kopf wird dabei der Strom WK entnommen, der über den Vorwärmer (60) überhitzt und anschließend in einer ersten und zweiten Verdichterstufe (61, 62) verdichtet und anschließend zur Beheizung des Seitenverdampfers STV eingesetzt wird. Der aus dem Seitenverdampfer erhaltene Strom wird über den Vorwärmer (60) zum Flashbehälter (66) geführt. Die gasförmige Phase wird vor den Vorwärmer (60) geführt. Die flüssige Phase wird teilweise mit einer ersten Pumpe (64) zurück zur Destillationskolonne DW1 (18) zurückgeführt. Die anderen beiden Teile werden mittels einer zweiten Pumpe (65) weitergeführt. Ein weiterer Teil der flüssigen Phase wird mit WK1 nach der Verdichtung (61, 62) vermischt. Ein weiterer Teil wird in einer weiteren Verdichterstufe (63), ggf. mit der selben Verdichtermaschine verdichtet und als WK2 zur Beheizung des Sumpfverdampfers eingesetzt. Anschließend wird der Strom ebenfalls zum Flashbehälter geführt. Die Verdichtungen (61, 62, 63) werden vorzugsweise in einer einzigen mehrstufigen Verdichtermaschine durchgeführt.

Die vorliegende Erfindung wird anhand des nachfolgenden Beispiels erläutert. Eine Einschränkung des Erfindungsgegenstands stellen diese Beispiele jedoch nicht dar.

### Beispiele

Für alle nachfolgenden Beispiele wurde ein MTBE-Strom von 35 t/h eingesetzt. Der MTBE-Strom hat die folgende Zusammensetzung: 97,5 wt% MTBE, 1wt% MeOH, 0,5 wt% MSBE (Methyl-sec-butyl-ether), 0,5 wt% TBA (tert.-Butanol), 0,25 wt% Wasser, 0,14 wt% i-Pentan, 0,1 wt% Diisobuten und Spuren von Leichtsiedern (600 ppm trans-2-Buten / 400 ppm cis-2-Buten).

Die für den Betrieb, der in den Beispielen dargestellten Anlagen notwendige Menge an Energie zur Spaltung von MTBE in die Hauptbestandteile Isobuten und Methanol MTBE wurde anhand einer Simulation mittels Aspen V12 errechnet. Die Stoffdaten und das kinetische Modell wurden durch Betriebsdaten und Betriebsversuche validiert.

### Beispiel 1 (nicht erfindungsgemäß)

Beispiel 1 wurde mit der in Fig. 1 beschriebenen Anlagenverschaltung durchgeführt. Die Kondensationswärme von 3 MW der Kolonnen DL (1) am Wärmeübertrager (3) wird in der Ausführungsform gemäß Fig. 1 nicht genutzt. Gleiches gilt für die Kondensationswärme am Wärmeübertrager (19) von 6 MW der Kolonne DW1 (18). Erfindungsgemäß werden beide Kondensationswärmen recycliert und mit Hilfe eines Wärmepumpen-Netzwerks aufgewertet. Als Arbeitsfluid wird n-Pentan bei 2,0 bar abs. in den Wärmeübertragern (3) und (19) verdampft. Es werden 155 t/h n-Pentan verdampft. Das Arbeitsfluid wird zunächst überhitzt (DT = 35 K) und dann einer mehrstufigen Verdichtung (41-1 und 41-2) zugeführt. Die Verdichterleistung beträgt 2,5 MW. Anschließend werden im Wärmeübertrager (30) in Summe 20 t/h 3 bar Dampf (DS2) erzeugt werden. Dieser 3 bar Dampf (DS2) wird zur Beheizung der Kolonnen DL (1), DW1 (18), DR (15) und DW2 (26) verwendet. Zusätzlich bleiben 4,7 t/h 3 bar Dampf übrig, die dem 3 bar Dampf, der aus der vorhandenen Dampferzeugung am Wärmeübertrager (8) zugeführt werden. Der 3 bar Dampf wird mit einer Dampfverdichtung über mehrere Stufen (51-1, 51-2 und 51-3) mit optionaler Zwischeneindüsung von Kondensat (nicht gezeigt) auf 16 bar (Strom DS3) gebracht, um die Kolonne DM (6) im Verdampfer (7) zu beheizen und in einer weiteren Stufe auf 40 bar gebracht um den vorhandenen Marlothermkreislauf über den Wärmeübertrager (35) zu beheizen. Hierzu ist ein Leistungseintrag des mehrstufigen Verdichters von 5 MW notwendig. Insgesamt werden 7,5 MW Strom eingesetzt.

### Beispiel 2 (erfindungsgemäß)

Beispiel 2 wurde mit der in Fig. 2 beschriebene Anlagenverschaltung durchgeführt. Durch die Brüdenverdichtung muss die Kolonne DW1 (18) nicht mehr mit Dampf beheizt werden. Die insgesamt benötigte Verdichterleistung verringert sich um 1,2 MW auf insgesamt 6,3 MW.

Die erfindungsgemäße Verschaltung hat gegenüber der alternativen, nicht erfindungsgemäßen Verschaltung also einen zusätzlichen Vorteil, dass damit noch mehr Energie eingespart werden kann.

## Patentansprüche

1. Verfahren zur Spaltung von MTBE, wobei das Verfahren zumindest die folgenden Schritte aufweist:
a) Bereitstellen eines Feedstroms, der MTBE enthält;
b) Leichtsiederabtrennung aus dem in Schritt (a) bereitgestellten Feedstrom in einer Destillationskolonne DL, wobei am Kopf ein Strom LK anfällt und der Sumpfstrom LS zum nachfolgenden Schritt (c) geführt wird;
c) Destillative Abtrennung des MTBE aus dem Sumpfstrom LS in mindestens einer Destillationskolonnen DM, wobei am Kopf von DM ein Strom K anfällt, der zumindest MTBE enthält, wobei der Strom K teilweise kondensiert wird, wodurch Energie auf einen Wärmeträger WA übertragen und ein Dampfstrom DS1 erzeugt wird und teilweise zur nachfolgenden Spaltungsreaktion in Schritt (d) geführt wird;
d) Durchführen der Spaltungsreaktion von MTBE mit dem Strom K in der Gasphase in mindestens einem Reaktor unter Verwendung eines heterogenen Katalysators, wodurch ein Reaktionsgemisch R erhalten wird, das zumindest Isobuten, Methanol, Wasser und MTBE enthält, wobei der Strom K vor dem Eintritt in den mindestens einen Reaktor und/oder der mindestens eine Reaktor mit einem Wärmeträgerfluid beheizt wird, das über einen Wärmeträgerfluidkreislauf bereitgestellt wird;
e) Destillation des Reaktionsgemischs R in mindestens einer Destillationskolonne DR, wobei ein Strom IB, der zumindest Isobuten enthält, und ein Strom M, der zumindest Methanol und Wasser enthält, anfallen;
f) Wasserabtrennung aus Strom M in einer einzigen Destillationskolonne DW1, wobei am Kopf der Destillationskolonne DW1 ein Strom WK, der zumindest Methanol enthält, und am Sumpf ein Strom, der zumindest Wasser enthält, anfällt, wobei der Strom WK einer ein- oder mehrstufigen Brüdenverdichtung unterworfen und dann zur Beheizung der Destillationskolonne DW1 eingesetzt wird;
g) Zumindest teilweises Kondensieren des Kopfstroms LK, wobei die Kondensationswärme dadurch genutzt wird, dass Energie vom Kopfstrom LK auf einen flüssigen oder gasförmigen Wärmeträger W übertragen wird, wodurch ein Wärmeträger W1 entsteht;
h) Zumindest teilweises Kondensieren des Reaktionsgemisches R, wobei die Kondensationswärme dadurch genutzt wird, dass Energie vom Reaktionsgemisch R auf einen flüssigen oder gasförmigen Wärmeträger W übertragen wird, wodurch ein Wärmeträger W2 entsteht;
i) Vermischen der beiden Wärmeträger W1 und W2 unter Erhalt des Wärmeträgers W3;
j) Verdichten mindestens eines Teils des Wärmeträgers W3, vorzugsweise des gesamten Wärmeträgers W3, wodurch ein verdichteter Wärmeträger W4 entsteht, der einen höheren Druck aufweist als der Wärmeträger W3; und
k) Übertragen von Energie vom verdichteten Wärmeträger W4 auf einen weiteren Wärmeträger WB übertragen wird, wodurch ein Dampfstrom DS2 erzeugt wird, der teilweise zur Beheizung der Destillationskolonne DL und der Destillationskolonne DR verwendet wird und teilweise mit dem Dampfstrom DS1 vermischt, unter Erhalt eines verdichteten Dampfstroms DS3 verdichtet und zur Beheizung der Destillationskolonne DM und nach weiterer Verdichtung unter Erhalt eines weiter verdichteten Dampfstroms DS4 zur Beheizung des Wärmeträgerfluidkreislaufs eingesetzt wird.

2. Verfahren nach Anspruch 1, wobei das in Schritt d) eingesetzte Wärmeträgerfluid Marlotherm^{®}, Hexan oder Wasser ist.

3. Verfahren nach Anspruch 1 oder 2, wobei der Wärmeträger W aus der Gruppe, bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt wird, vorzugsweise Wasser, Methanol, Ethanol, Propanol, n-Pentan, n-Butan, n-Hexan, n-Propan oder Ammoniak, besonders bevorzugt n-Pentan ist.

4. Verfahren nach einem der vorhergehenden Ansprüche, wobei der in Schritt c) erzeugte Dampfstrom DS1 und der in Schritt k) erzeugte Dampfstrom DS2 jeweils ein Wasserdampfstrom sind.

5. Verfahren nach einem der vorhergehenden Ansprüche, wobei der Dampfstrom DS2 in Schritt k) einen Druck im Bereich von 2 bis 5 bar aufweist.

6. Verfahren nach einem der vorhergehenden Ansprüche, wobei der Dampfstrom DS3 nach der Verdichtung einen Druck im Bereich von 8 bis 20 bar aufweist und anschließend zur Beheizung der Destillationskolonne DM eingesetzt wird.

7. Verfahren nach einem der vorhergehenden Ansprüche, wobei der Dampfstrom DS4 nach der Verdichtung einen Druck im Bereich von 21 bis 55 bar aufweist und anschließend zur Beheizung des Wärmeträgerfluidkreislaufs eingesetzt wird.

8. Verfahren nach einem der vorhergehenden Ansprüche, wobei der Wärmeträger W3 in zwei Verdichterstufen V1 und V2 verdichtet wird, wobei in der ersten Verdichtung ein verdichteter Wärmeträger W3.1 entsteht, der einen höheren Druck als W3 aufweist und in der zweiten Verdichtung der verdichtete Wärmeträger W4 entsteht, der einen höheren Druck als W3.1 und als W3 aufweist.

9. Verfahren nach Anspruch 8, wobei der Wärmeträger W4 bei der Energieübertragung in Schritt k) zumindest teilweise kondensiert.

10. Verfahren nach einem der vorhergehenden Ansprüche, wobei die Destillationskolonne DW1 einen Seitenverdampfer STV und einen Sumpfverdampfer SV aufweist.

11. Verfahren nach Anspruch 10, wobei der Strom WK verdichtet wird, wodurch der Strom WK1 entsteht, und der Strom WK1 anschließend zur Beheizung im Seitenverdampfer STV eingesetzt wird.

12. Verfahren nach Anspruch 11, wobei der Strom WK1 nach der Beheizung im Seitenverdampfer STV zu einem Flashbehälter geführt wird, wobei der Strom WK1 vor dem Eintritt in den Flashbehälter vorzugsweise zur Vorwärmung des Stroms WK vor der Verdichtung eingesetzt wird.

13. Verfahren nach Anspruch 12, wobei im Flashbehälter eine gasförmige Phase, die mit dem Strom WMK vor der Vorwärmung vermischt wird, und eine flüssige Phase anfällt, die zumindest teilweise zurück zur Destillationskolonne DW1 und teilweise unter Erhalt des Stroms WK2 verdichtet und anschließend zur Beheizung im Sumpfverdampfer SV eingesetzt wird und optional teilweise mit dem Strom WK1 vor der Beheizung des Seitenverdampfers vermischt wird.

14. Verfahren nach Anspruch 13, wobei der Strom WK2 nach der Beheizung des Sumpfverdampfers zum Flashbehälter geführt wird.

15. Verfahren nach Anspruch 13 oder 14, wobei das Zurückführen der flüssigen Phase zur Destillationskolonne DW1 und/oder das Zuführen der flüssigen Phase zur Verdichtung zu WK2 mittels einer Pumpe erfolgt.
